# EUROPÄISCHE PATENTSCHRIFT

(11) **EP 0 582 226 B1**
(45) Veröffentlichungstag und Bekanntmachung des Hinweises auf die Patenterteilung: **01.10.1997**
(21) Anmeldenummer: 93112210.5
(22) Anmeldetag: 30.07.1993
(51) Int. Cl.: A61B 5/14

(54) **Stechgerät**
Lancet device
Dispositif de piqûre

(30) Priorität: 03.08.1992 PL 295552
(43) Veröffentlichungstag der Anmeldung: 09.02.1994
(73) Patentinhaber: PRZEDSIEBIORSTWO ZAGRANICZNE HTL, 03-230 Warszawa (PL)
(72) Erfinder: Czernecki, Andrzej, 03-934 Warszawa (PL); Firchal, Dariusz, 02-930 Warszawa (PL); Krolikowski, Marek, 00-350 Warszawa (PL)
(74) Vertreter: Hennicke, Albrecht, Dipl.-Ing.

(56) Entgegenhaltungen:
- EP-A- 0 081 665
- EP-A- 0 255 338
- US-A- 4 889 117

## Beschreibung

Die Erfindung betrifft ein Stechgerät zum Ritzen der Haut eines Patienten, insbesondere für die Entnahme von Blutproben aus dem Finger, dem Ohr oder der Ferse des Patienten für diagnostische Zwecke.

Zum Anstechen der Haut am Finger eines Patienten und zur Entnahme einer Blutprobe ist eine Lanzette bekannt (US-A-4 889 117), die aus einer langen und steifen Stange besteht, die an einem Ende eine Punktierspitze in Form einer Subkutan-Nadel aufweist. An ihrem anderen Ende ist die Stange mit einer Kapsel verbunden. Die Stange ist auf ihrer ganzen Lange in einer Hülse gleitend gelagert, die teilweise in die Kapsel eingreift. Zwischen der Hülse und dem Boden der Kapsel ist eine Feder angeordnet. Wenn man die Öffnung der Hülse gegen den Finger des Patienten hält und auf die Kapsel drückt, wird die Hülse veranlaßt, die Federkraft zu überwinden und sich um einen vorbestimmten Abstand in Richtung auf den Boden der Kapsel zu bewegen. Diese Bewegung reicht aus, um mit der an der Stange befestigten Punktierspitze die Haut des Fingers des Patienten zu ritzen. Nach dem Gebrauch der Lanzette kehrt die Punktierspitze vollständig in die Hülse zurück und ein nach innen vorspringendes Kapselelement macht es unmöglich, die Lanzette wieder zu verwenden.

Es ist ferner eine Lanzette der eingangs näher erläuterten Art bekannt (EP-A-0 255 338), bei der der die Punktierspitze tragende Kolben mit einer Grundplatte versehen ist, die auf einer Ringschulter mit Klebstoff oder einer Heißsiegelmasse festgeklebt ist. Diese Grundplatte wird von ihrem Sitz abgerissen, wenn ein in einer besonderen Kappe angeordnetes Betätigungsorgan auf den Kolben einwirkt. Beim Abreißen der Grundplatte können Klebstoffreste zwischen den Plattenrand und die Innenwandung der Hülse geraten und eine einwandfreie Axialbewegung des Kolbens mit der Punktierspitze verhindern, so daß diese hängenbleiben oder schief aus dem Gerät austreten kann. Eine einwandfreie Funktion ist dann nicht gewährleistet.

Bei einer anderen Ausführungsform der bekannten Lanzette ist die Punktierspitze in einer Grundplatte befestigt, die mit radial sich erstreckenden Armen eine Öffnung in der Hülse übergreift. Diese Radialarme weichen elastisch nach oben aus, wenn die Grundplatte mit ihrer Punktierspitze von einem Betätigungsorgan durch die Öffnung gedrückt wird, und sie gleiten, die Grundplatte mit der Punktierspitze führend, an der Innenwandung der Hülse entlang, wenn die Punktierspitze von einer Feder durch eine Öffnung im unteren Boden der Hülse gedrückt wird.

Bei dieser bekannten Ausführung ist eine beträchtliche Kraft notwendig, um die Grundplatte mit der Punktierspitze durch die obere Öffnung in der Hülse zu drücken, und es bedarf einer sehr kräftigen Feder, um die Punktierspitze aus der Hülse herausschnellen zu lassen. Vor allem aber ist ein vollständiges Zurückgleiten der Punktierspitze in die Hülse in Frage gestellt, da die an der Hülsenwandung entlanggleitenden Radialarme nach dem Richtungswechsel nicht mit einem abgeschrägten, sondern einem scharfen Rand an der Innenwandung der Hülse entlanggleiten und hierdurch stark abgebremst werden.

Bei einer anderen bekannten Lanzette (EP-A-0081 665) ist zwischen einem Lanzettenschaft mit der Punktierspitze und einem Betätigungsorgan ein Kolben in einer Hülse zwischen zwei Federn axial beweglich angeordnet, der beim Spannen der einen Betätigungsfeder durch ein von außen zu betätigendes Auslöseelement festgehalten wird. Nach dem Auslösen des Kolbens treibt dieser über die Rückstellfeder die Lanzette mit der Punktierspitze nach außen, die anschließend von der Rückstellfeder wieder eingezogen wird.

Bei diesem bekannten Stechgerät kann die Lanzette mit der Punktierspitze zweimal benutzt werden, wenn der Kolben mit dem Betätigungselement schon gelöst wird, bevor der Spannknopf für die Vorschubfeder vollständig niedergedrückt und im Gehäuse verriegelt wurde.

Aufgabe der Erfindung ist es, den bekannten Punktiergeräten ein weiteres Gerät an die Seite zu stellen, das eine sehr einfache, gedrungene Bauart hat, nur einmal verwendet werden kann und die handhabende Person sicher davor schützt, mit der Blutprobe des Patienten in Berührung zu kommen.

Diese Aufgabe wird mit einem Stechgerät nach der Erfindung dadurch gelöst, daß der Kolben an seinem Außenumfang nur begrenzt haltbare Flügel aufweist, die sich auf einer Schulter der Hülse in deren Axialrichtung abstützen und daß der Kolben aus der Hülse durch eine vorgespannte Druckfeder herausbewegbar ist, die zwischen dem Kolben und einem axial beweglichen Druckorgan angeordnet ist, mit dem auf den Kolben eingewirkt werden kann, um dessen Flügel zu zerstören und daß eine Rückstellfeder vorgesehen ist, die den Kolben und seine Punktierspitze in die Hülse zurückbewegt.

Diese Ausgestaltung hat den Vorteil, daß die Flügel für den Kolben zerstört werden, wenn dieser zum Ausfahren der Punktierspitze in die Hülse gedrückt wird. Die Vorschubfeder kann deshalb nicht ein zweites Mal gespannt werden und die Punktierspitze aus der Öffnung im Boden der Hülse heraustreiben. Das Gerät kann deshalb nach der Zerstörung der Flügel am Kolben nicht wiederverwendet werden, so daß eine Kontamination und die Gefahr der Übertragung von Blutkrankheiten ausgeschlossen ist.

Weitere Merkmale und Vorteile der Erfindung ergeben sich aus der nachfolgenden Beschreibung und den Zeichnungen, in denen eine bevorzugte Ausführungsform der Erfindung an einem Beispiel näher erläutert wird. Es zeigt:
- Fig. 1: ein Stechgerät nach der Erfindung vor seinem Gebrauch in einem Längsschnitt und
- Fig. 2: das Stechgerät nach Fig. 1 nach seinem Gebrauch ebenfalls in einem Längsschnitt.

Das in den Zeichnungen dargestellte Stech- oder Punktiergerät, das zum Ritzen der Haut für die Entnahme von Blutproben für diagnostische Zwecke dient, besteht aus einer Hülse 1 und einer Druckkappe 2, die am einen Ende der Hülse 1 angeordnet ist. Das zweite Ende der Hülse 1 endet in einem Boden 3, der ein Loch 4 aufweist.

Im Inneren der Hülse 1 ist ein Kolben 5 gleitend gelagert, der an seinem der Druckkappe 2 zugewandten Ende einen Drücker 6 trägt und auf seiner dem Loch 4 im Boden 3 der Hülse 1 zugewandten Ende mit einer Punktierspitze 7 versehen ist.

Zwischen der Innenfläche der Druckkappe 2 und dem Kolben 5 ist im Inneren der Hülse 1 eine Druckfeder 9 angeordnet, während zwischen dem Kolben 5 und dem Boden 3 der Hülse 1 eine Rückstellfeder 10 untergebracht ist. Der Kolben 5 ist an seinem Außenumfang mit nur begrenzt haltbaren Haltemitteln 11 in Form von angeformten Flügeln versehen, die sich auf einer Schulter 12 der Hülse 1 abstützen.

Die Wirkungsweise des Gerätes ist folgende:

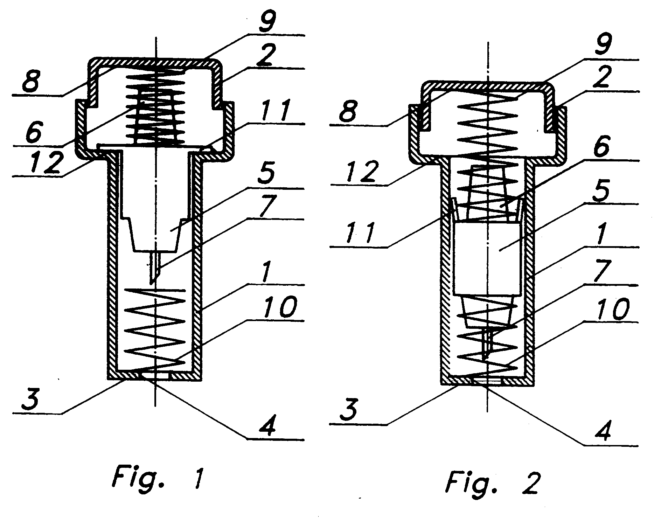

Vor dem Gebrauch haben die einzelnen Teile des Gerätes die in Fig. 1 dargestellte Lage. Unter dem von der Druckfeder 9 ausgeübten Druck werden die Haltemittel oder Flügel 11 des Kolbens 5 gegen die Schulter 12 der Hülse gepreßt und halten den Kolben 5 mit seiner Punktierspitze 7 in seiner ersten, stabilen Lage. Bei einem Druck auf die Druckkappe 2 wird zunächst die Druckfeder 9 zusammengedrückt, bis die Innenfläche 8 der Druckkappe 2 gegen den Drücker 6 des Kolbens 5 stößt. Wird dann weiter auf die Druckkappe 2 gedrückt, brechen die Ansätze oder Flügel 11 vom Kolben 5 ab, so daß die Druckfeder 9 den Kolben 5 nach unten treibt und die Punktierspitze 7 das Loch 4 im Boden 3 der Hülse 1 durchdringt und in die zu punktierende Hautoberfläche einsticht. Danach drückt die Rückstellfeder 10 den Kolben 5 mit seiner Punktierspitze 7 zurück, die hierdurch wieder eine stabile Lage im Inneren der Hülse 1 einnimmt, wo der Kolben 5 zwischen der Druckfeder 9 und der Rückstellfeder 10 im Inneren der Hülse 1 gehalten wird. Da beim Niederdrücken der Druckkappe 2 der Drücker 6 nicht mehr erreicht werden kann und auch die hierbei erzeugte Vorspannung der Druckfeder 9 nicht mehr ausreicht, um den Kolben 5 entgegen der Wirkung der Rückstellfeder 10 soweit zu verschieben, daß die Punktierspitze 7 aus dem Loch 4 im Boden 3 in der Hülse 1 heraustritt, ist es unmöglich, das Stechgerät wieder zu verwenden. An der Punktierspitze 7 haftengebliebenes Blut kann deshalb auch keine Infektionsquelle sein.

Die Erfindung ist nicht auf das dargestellte und beschriebene Ausführungsbeispiel beschränkt, sondern es sind mehrere Änderungen und Ergänzungen möglich, ohne den Rahmen der Erfindung zu verlassen. Beispielsweise könnte der Drücker oder das Druckelement 6 auch an dem Druckorgan 2 befestigt sein, welches auch etwas anders ausgebildet sein kann. Anstelle der Flügel 11 kann der Kolben 5 auch mit anderen Haltemitteln, beispielsweise vorspringenden Nasen od.dgl. versehen sein, die in entsprechende Ausnehmungen in der Hülsenwand eingreifen und abgeschert werden, wenn auf den Kolben eine Kraft in Axialrichtung ausgeübt wird.

## Patentansprüche

1. Stechgerät zum Ritzen der Haut eines Patienten, insbesondere für die Entnahme von Blutproben für diagnostische Zwecke, mit einer Hülse (1) und einem darin axial beweglichen, mit einer Punktierspitze (7) versehenen Kolben (5), der durch Federmittel (6 bzw. 10) mit seiner Punktierspitze (7) aus der Hülse (1) heraus- und/oder in diese zurückbewegbar ist, **dadurch gekennzeichnet, daß** der Kolben (5) an seinem Außenumfang nur begrenzt haltbare Flügel (11) aufweist, die sich auf einer Schulter (12) der Hülse (1) in deren Axialrichtung abstützen und daß der Kolben (5) aus der Hülse (1) durch eine vorgespannte Druckfeder (9) herausbewegbar ist, die zwischen dem Kolben (5) und einem axial beweglichen Druckorgan (2) angeordnet ist, mit dem auf den Kolben (5) eingewirkt werden kann, um dessen Flügel (11) zu zerstören und daß eine Rückstellfeder (10) vorgesehen ist, die den Kolben (5) und seine Punktierspitze (7) in die Hülse (1) zurückbewegt.

2. Gerät nach Anspruch 1, **dadurch gekennzeichnet, daß** die Hülse (1) an ihrem einen Ende einen geschlossenen Boden (3) mit einem Loch (4) für den Durchgang der Punktierspitze (7) des Kolbens (5) aufweist und daß die Rückstellfeder (10) zwischen dem Kolben (5) und dem geschlossenen Boden (3) der Hülse (1) angeordnet ist.

3. Gerät nach Anspruch 1 oder 2, **dadurch gekennzeichnet, daß** zwischen dem Druckorgan (2) und dem Kolben (5) ein Druckelement (6) angeordnet ist, das von der Druckfeder (9) umgeben wird.

4. Gerät nach Anspruch 3, **dadurch gekennzeichnet, daß** das Druckelement (6) am Kolben (5) befestigt ist.

5. Gerät nach Anspruch 3, **dadurch gekennzeichnet, daß** das Druckelement (6) an dem Druckorgan (2) befestigt ist.

6. Gerät nach einem der Ansprüche 1 bis 5, **dadurch gekennzeichnet, daß** das Druckorgan (2) als eine an der Hülse (1) geführte Druckkappe ( ) ausgebildet ist, die das andere Ende der Hülse (1) verschließt.

## Claims

1. Pricking device to nick the skin of a patient, especially for taking blood samples for diagnostic purposes, with a shell (1), and a piston (5) axially movable therein and provided with a puncturing tip (7) and movable with its puncturing tip (7) outwardly from and retractably into the shell (1) by spring means (6 or 10), characterised in that the piston (5) has, at its outer periphery, wings (11) of only limited strength which bear against a shoulder (12) of the shell (1) in the axial direction of the latter, and that the piston (5) is outwardly movable from the shell (1) by a pre-loaded compression spring (9) which is arranged between the piston (5) and an axially movable pressure member (2) that can act upon the piston (5) in order to destroy its wings (11), and that a return spring (10) is provided which retracts the piston (5) and its puncturing tip (7) back into the shell (1).

2. Device according to claim 1, characterised in that the shell (1) at one end has a closed base (3) with a hole (4) for the passage of the puncturing tip (7) of the piston (5), and that the return spring (10) is arranged between the piston (5) and the closed base (3) of the shell (1).

3. Device according to claim 1 or 2, characterised in that a pressure element (6) is arranged between the pressure member (2) and the piston (5) and is surrounded by the compression spring (9).

4. Device according to claim 3, characterised in that the pressure element (6) is secured to the piston (5).

5. Device according to claim 3, characterised in that the pressure element (6) is secured to the pressure member (2).

6. Device according to one of claims 1 to 5, characterised in that the pressure member (2) is formed as a pressure cap ( ) guided on the shell (1) and closes off the other end of the shell (1).

## Revendications

1. Appareil produisant une piqûre destinée à égratigner la peau d'un patient, en particulier pour le prélèvement d'échantillons sanguins à des fins de diagnostic, comprenant une douille (1) et un piston (5) mobile axialement dans cette dernière, qui est muni d'une pointe de ponction (7) et qui peut sortir de la douille (1) et/ou se rétracter dans cette dernière avec sa pointe de ponction (7) grâce à des éléments-ressorts (6 et 10), caractérisé en ce que le piston (5) présente, sur sa périphérie extérieure, des ailettes (11) qui ne sont conservées que de manière limitée et qui prennent appui sur un épaulement (12) de la douille (1), dans la direction axiale de cette dernière, en ce que le piston (5) peut être sorti de la douille (1) par un ressort de compression (9) en précontrainte disposé entre le piston (5) et un organe de pression (2) mobile axialement et permettant une intervention sur le piston (5) en vue de détruire son ailette (11), et en ce qu'il est prévu un ressort de rappel (10) qui provoque la rétraction du piston (5) et de sa pointe de ponction (7) dans la douille (1).

2. Appareil selon la revendication 1, caractérisé en ce que la douille (1) présente, à une de ses extrémités, un fond fermé (3) percé d'un trou (4) pour le passage de la pointe de ponction (7) du piston (5), et en ce que le ressort de rappel (10) est disposé entre le piston (5) et le fond fermé (3) de la douille (1).

3. Appareil selon la revendication 1 ou 2, caractérisé en ce qu' un élément de pression (6) entouré par le ressort de compression (9) est disposé entre l'organe de pression (2) et le piston (5).

4. Appareil selon la revendication 3, caractérisé en ce que l'élément de pression (6) est fixé au piston (5).

5. Appareil selon la revendication 3, caractérisé en ce que l'élément de pression (6) est fixé à l'organe de pression (6).

6. Appareil selon l'une des revendications 1 à 5, caractérisé en ce que l'organe de pression (6) est réalisé sous forme de capuchon de pression ( ) guidé sur la douille (1), qui obture l'autre extrémité de la douille (1).
